Europäisches Patentamt

European Patent Office

Office européen des brevets

(11) Veröffentlichungsnummer: **0 252 302**
**A1**

(12) **EUROPÄISCHE PATENTANMELDUNG**

(21) Anmeldenummer: 87108169.1

(22) Anmeldetag: 05.06.87

(51) Int. Cl.³: **C 12 N 15/00**
**//A61K39/245, G01N33/569**

(30) Priorität: 12.06.86 DE 3619720

(43) Veröffentlichungstag der Anmeldung:
13.01.88 Patentblatt 88/2

(84) Benannte Vertragsstaaten:
AT BE CH DE ES FR GB GR IT LI LU NL SE

(71) Anmelder: BEHRINGWERKE Aktiengesellschaft
Postfach 1140
D-3550 Marburg 1(DE)

(72) Erfinder: Mach, Michael, Dr.
Geschwister-Scholl-Strasse 10
D-8530 Erlangen(DE)

(72) Erfinder: Utz, Ursula
Hindenburgstrasse 59
D-8530 Erlangen(DE)

(72) Erfinder: Fleckenstein, Bernhard, Prof.
Nr.93
D-8551 Schlaifhausen(DE)

(74) Vertreter: Meyer-Dulheuer, Karl-Hermann, Dr. et al,
HOECHST Aktiengesellschaft Zentrale Patentabteilung
Postfach 80 03 20
D-6230 Frankfurt/Main 80(DE)

(54) Hauptglykoprotein des menschlichen Cytomegalovirus, seine Herstellung und Verwendung.

(57) Das HindIII-F-Fragment aus dem Genom des humanen Cytomegalovirus (HCMV), Stamm Ad 169, enthält einen offenen Leserahmen, der für immunogene Proteine des Glykoprotein A-Komplexes kodiert. Durch Expression gewonnene derartige immunogene Produkte eignen sich als Diagnostika und zur Herstellung von Impfstoffen.

EP 0 252 302 A1

Hauptglykoprotein des menschlichen Cytomegalovirus,
seine Herstellung und Verwendung

Das Hauptglykoprotein in der Virushülle des humanen Cytomegalovirus (HCMV) weist ein Molgewicht von ca. 58 kd auf
(gp 58). Es ist ein Bestandteil des Glykoprotein A-Komplexes, der mit Hilfe von monoklonalen Antikörpern gefällt
werden kann und aus mindestens 3 Glykoproteinen mit den
geschätzten Molgewichten von 130 kd, 95 kd und 58 kd besteht (L. Pereira et al., Virology 139 (1984) 73 - 86).
Es zeigte sich jedoch, daß die bisher bekannten monoklonalen Antikörper gegen den Glykoprotein A-Komplex denaturierte
Proteine nicht erkennen und somit ungeeignet sind, mit
Hilfe einer Genbank gewonnene Klone zu identifizieren,
die gp 58 oder Teile davon exprimieren.

Die Erfindung bezieht sich auf immunogene Proteine aus dem
Glykoprotein A-Komplex, insbesondere gp 58, auf Verfahren
zur Herstellung solcher Proteine und deren Verwendung als
Diagnostica bzw. zur Herstellung von Antikörpern und Impfstoffen. Bevorzugte Ausgestaltungen der Erfindung in ihren
verschiedenen Aspekten sind im folgenden näher erläutert
bzw. in den Patentansprüchen definiert.

Es wurde gefunden, daß man mit einem monospezifischen Kaninchen-Antiserum gegen gp 58 die gewünschten Klone aus
einer HCMV-cDNA-Genbank identifizieren kann. Hierzu wurde
eine Probe des gesamten viralen Proteins einer präparativen
SDS-Polyacrylamid-Gelelektrophorese unterworfen und die
einzelnen Proteinbanden mit Hilfe des Farbstoffs Coomassie
Brillant-Blau sichtbar gemacht. Die Region, die dem Molekulargewichtsbereich 56 bis 58 kd entsprach, wurde herausgeschnitten, das Protein extrahiert und zur Immunisierung von Kaninchen verwendet. Nach der ersten Applikation
wurden im Abstand von 3 Wochen Auffrischungsimpfungen im
Laufe von 7 Monaten verabreicht. Das gewonnene Antiserum

zeige im "Western Blot"-Test, daß ein einziges Protein vom 58 kd Größe erkannt wird. Wurde dieses Serum auf infizierte Zellen angewandt, so ergab sich keine besonders bevorzugte Reaktion für das 58 kd-Protein; statt dessen reagierte das Serum auch mit Proteinen vom Molgewicht 95 kd, 130 kd und 160 kd. Das Serum reagierte jedoch nicht mit Extrakten uninfizierter Zellen. Dieses Serum wurde für das "Screening" der cDNA-Genbank eingesetzt.

Zur Herstellung der Genbank wurde aus mit HCMV, Stamm Ad 169, infizierten menschlichen Vorhaut-Fibroblastenzellen 96 bis 120 Stunden nach der Infektion die $poly(A)^+$-RNA isoliert, in ds-DNA überführt und ohne Größenfraktionierung in den handelsüblichen Phagen-Expressionsvektor gt11 eingefügt. Hierzu wurde der Vektor mit EcoRI gespalten und mit alkalischer Phosphatase (aus Kälberdarm) behandelt, um die intramolekulare Religation zu unterdrücken. Durch Anfügen von EcoRI-Linkern wurde die cDNA zwischen die Phagenarme eingefügt und in vitro verpackt. Aus 100 ng ds-cDNA wurde so eine Genbank erhalten, die etwa $5 \cdot 10^5$ unabhängige Rekombinanten und 18 % Wildtyp-Phagen enthielt.

Das "Screening" der Genbank erfolgte nach der Methode von R. A. Young und R. W. Davis, Proc. Natl. Acad. Sci. USA 80 (1983) 1194 - 1198, jedoch mit der Abwandlung, daß Meerrettich-Peroxidase an Protein A gekoppelt wurde und 4-Chlor-1-naphthol als Detektionssystem diente, unter Einsatz der vorstehend beschriebenen monospezifischen Kaninchen-Antikörper.

Von 150 000 untersuchten Plaques wurden 15 positive Signale erhalten. Ein Klon mit einer Insertion von etwa 400 Basen wurde für die weitere Charakterisierung ausgewählt; er erhielt die Bezeichnung UM-2.

- 3 -

0252302

Der E. coli-Stamm Y 1089 wurde mit dem rekombinanten Phagen infiziert und die Synthese des ß-Galaktosidase-Proteins durch Zugabe von Isopropylthiogalaktosid (IPTG) induziert. Hierbei wurde ein 119 kd-Protein gebildet, das weder in nichtinfizierten Zellen noch in infizierten, aber nichtinduzierten Zellen gefunden wurde. Im "Western Blot"-Test wurde dieses Protein spezifisch von dem Kaninchen-Anti-gp 58-Serum erkannt. Das Anti-gp 58-Serum reagierte nur mit einem einzigen Protein in UM-2-infizierten, induzierten Zellen, erkannte jedoch weder Proteine in nicht-infizierten oder nichtinduzierten infizierten Zellen. Es ergibt sich somit, daß der rekombinante Klon UM-2 ein Fusionsprotein mit einem HCMV-Glykoprotein-Anteil synthetisiert.

Die cDNA-Insertion von 400 bp wurde nun verwendet, um das Gen für gp 58 im Virus-Genom zu lokalisieren: Hierfür wurde die cDNA-Insertion von 400 bp mit 8 Cosmid-Klonen hybridisiert, die das gesamte Genom von HCMV umspannen (B. Fleckenstein et al., Gene 18 (1982) 39 - 46). Das Cosmid pCM 1029, das die Fragmente HindIII-D und -F enthält, hybridisierte mit der cDNA. Eine eingehendere "Southern Blot"-Analyse dieser Region begrenzte das HCMV-DNA-Fragment auf ein 1,3 kb EcoRI-BamHI-Fragment, das im HindIII-F-Fragment lokalisiert ist, und zwar benachbart zum D-Fragment.

Die Translation einer 400 bp cDNA-Insertion, zusätzlich zu dem offenen Leserahmen der ß-Galaktosidase, sollte ein Fusionsprotein von etwa 130 kd Größe ergeben. Das aus dem Klon UM-2 erhaltene Fusionsprotein ist offensichtlich kleiner. Wie jedoch die "Western Blot"-Daten und die Hybridisierung mit einem Fragment der HCMV-DNA unter stringenten Bedingungen anzeigen, enthält dieser Klon HCMV-Sequenzen. Offensichtlich enthält also die Insertion ein Stop-Signal innerhalb der cDNA. Da cDNA vom 3'-Ende der mRNA her synthetisiert wird und somit möglicherweise 3'-"untranslated regions" enthält, wurde zur Bestätigung die cDNA-Insertion orientierungsabhängig subkloniert. Hierzu wurde ein 1,3-kb KpnI-EcoRI-Fragment in den handelsüblichen Vektor M13mp19 eingefügt, wodurch die EcoRI-Schnittstelle, die an der ß-Galaktosidase-HCMV-Übergangsstelle gelegen ist, an den Anfang der Sequenz zu liegen kommt. Es zeigte sich, daß die Sequenz tatsäch 27 Basen unterhalb der ß-Galaktosidase-HCMV-Schnittstelle ein Stop-Kodon aufweist.

Der gleiche M13-Klon wurde zur Bestimmung der Transkriptionsrichtung verwendet. Hierzu wurde er in Form von Einzelsträngen an Einzelstrang-DNA des EcoRI-BamHI-Fragments hybridisiert, das in beiden Orientierungen in den Bakteriophagen M13 kloniert war. Es ergab sich, daß die für gp 58 kodierende mRNA in Richtung vom HindIII-D- zum -F-Fragment umgeschrieben wird.

Hieraus folgt, daß das für gp 58 kodierende Gen am 3'-Ende des offenen Leserahmens liegt, der für den Vorläufer des Gruppe A-Glykoprotein-Komplexes von HCMV kodiert. Da der nichtglykosylierte Vorläufer des Gruppe A Glykoproteins nach Pereira, a.a.O., ein 95 kd Polypeptid ist, ergibt sich, daß das dominante Oberflächen-Glykoprotein von HCMV durch proteolytische Spaltung entsteht und das Carboxyterminale Ende des primären Translationsprodukts darstellt. "Northern Blot"-Analysen ergaben verschiedene Größenklassen von "späten" RNA-Molekülen zwischen 2,5 und 8 kb. Der ganz überwiegend auftretende RNA-Typ war ein diskretes 4 kb Transkript.

Zur Herstellung von gp 58 und antigenwirksamen Teilen davon kann man nun einerseits in Kenntnis der Lokalisierung des Gens geeignete Restriktionsfragmente aus dem kartierten Virus-Genom auswählen oder anderseits mit Hilfe des 400 bp cDNA-Fragments die Genbank einem erneuten "Screening" unterziehen und so N-terminal verlängerte Klone identifizieren.

Ein geeignetes Restriktionsfragment aus dem Virus-Genom ist das HindIII-F-Fragment des HCMV, Stamm Ad 169. In diesem Fragment befindet sich eine EcoRV-Stelle etwa 700 bp unterhalb der singulären EcoRI-Stelle. Diese EcoRV-Stelle liegt ca. 40 bp unterhalb des Stop-Codons.

Zur Gewinnung eines immunogenen Peptids kann man beispielsweise ein 1,3 kb PstI-EcoRV-Fragment isolieren und in geeignete pro- und eukaryontische Expressionsvektoren inserieren. Auf diesem DNA-Fragment liegt die komplette codierende Sequenz für gp 58.

Weiterhin kann man ein 3,1 kb-PstI-EcoRV-Fragment aus dem HindIII-F-Fragment isolieren und zur Expression bringen. Dieser DNA-Abschnitt codiert auch Vorläufer-Proteine.

Auf klassischem Wege isoliertes oder rekombinant hergestelltes gp 58 oder immunogene gp 58-Fragmente können für diagnostische Zwecke verwendet werden, z.B. im ELISA. Dies gilt insbesondere, da gp 58 zu den wenigen Strukturproteinen zählt, die regelmäßig durch Patienten-Antikörper erkannt werden.

Weiterhin eignet sich gp 58 als Bestandteil eines Impfstoffes gegen HCMV, da dieses Hüllprotein die Synthese spezifischer, neutralisierender Antikörper im infizierten Wirt veranlaßt.

Beispiel 1

Das Plasmid pCM 5004 (R. Rüger et al., J. Gen. Virology 65 (1984) 1351 - 1364) enthält das EcoRI-M-Fragment aus dem Genom des HCMV, Stamm Ad 169, im Vektor pACYC184

- 6 -

0252302

(A.C.Y. Chang et al., J. Bacteriol. 134 (1978) 1141 - 1156).
Aus pCM 5004 wurde durch Verdauung mit EcoRI und BamHI ein
1,4 kb-Fragment erhalten. Dieses Fragment enthält etwa
800 bp von der EcoRI-Schnittstelle entfernt das Stop-Codon
des offenen Leserahmens, der für den Vorläufer des Glycoprotein A-Komplexes codiert.

Das Fragment wurde unter Bildung stumpfer Enden aufgefüllt
und in drei Expressionsvektoren cloniert, die die drei möglichen offenen Leserahmen repräsentieren. Diese drei Vektoren pUR 278, 288 und 289 (U. Rüther et al., EMBO Journal 2
(1983) 1791 - 1794) waren zuvor mit BamHI verdaut worden,
die Schnittstellen auf stumpfe Enden aufgefüllt und mit
Kälberdarmphosphatase desphosphoryliert worden. Die Insertion erfolgte hier an das Ende des lacZ-Gens, so daß bei
einer der drei Plasmidkonstruktionen ein Fusionsprotein
codiert ist, das ein Hybrid aus ß-Galactosidase und gp 58
bzw. einen Teil desselben darstellt.

Die Hybridplasmide wurden in den handelsüblichen E. coli-
Stamm NF 1829 transformiert, der eine lac i$^q$-Mutation
besitzt. Die Transformanten wurden in LB-Kulturmedium
unter Zusatz von Ampicillin und Kanamycin bei 37°C angezogen. Bei Erreichen der logarithmischen Wachstumsphase
wurde die Synthese des Fusionsproteins durch Zugabe von
Isopropyl-ß-D-thiogalacto-pyranosid (IPTG) bis zu einer
Endkonzentration von 2 mM induziert. Nach weiteren zwei
Stunden bei 37°C wurden die Zellen geerntet und durch 3 Minuten Kochen in SDS-Proteinpuffer lysiert.

Das Proteingemisch wurde über ein 8 %iges Polyacrylamidgel
aufgetrennt (pro Gelspur etwa 500 μl). Das resultierende
Fusionsprotein eines Klones zeigte eine Größe von etwa 145 kd.
Es reagierte im "Western blot"-Test mit einem monospezifischen Serum gegen das gp 58. Proteine aus plasmidfreien
oder nichtinduzierten Zellen bzw. aus induzierten Zellen, die die zugrundeliegenden Vektoren ohne HCMV-DNA enthielten, wurden von diesem Serum nicht erkannt.

Der rekombinante Klon synthetisiert somit ein Fusionsprotein mit einem HCMV-Anteil. Dieser HCMV-Anteil entspricht dem Carboxyterminus von gp 58 und besitzt eine Länge von etwa 270 Aminosäuren.

Analog hierzu kann man ein 900 bp PstI-Fragment exprimieren, das mit der DNA in dem vorstehend beschriebenen Klon um etwa 300 bp überlappt und in das EcoRI-P-Fragment reicht.

Analog kann man auch verschieden große HincII-Fragmente aus dem EcoRI-P-Fragment oder ein 870 bp EcoRV-Fragment aus dem EcoRI-P+M-Fragment einsetzen.

## Beispiel 2

Aus dem HCMV Hind III-F-Fragment wurde das ca. 5000 bp große Hind III-Bam HI-Fragment isoliert und in pUC 18, das mit Hind III und Bam HI verdaut worden war, ligiert. Aus diesem neuen Plasmid pBUM 5801 wurde das ca. 900 bp große PstI-Fragment isoliert und in pEX1, das mit PstI linearisiert worden war, kloniert. Das neue Plasmid pUU 5813e codiert somit für ein Fusionsprotein von ca. 150 000 d , bestehend aus ß-Galactosidase und ca. 90 % des prozessierten gp 58. Dieses durch pUU 5813 e codierte Fusionsprotein wurde in E.coli POP 2136 exprimiert und Gesamtzellextrakt auf Polyacrylamidgelen aufgetrennt. In der nachfolgenden Western blot-Analyse konnte nachgewiesen werden, daß HCMV-positive Patientenseren und auch HCMV neutralisierende monoklonale Antikörper mit dem Fusionsprotein reagieren, nicht aber mit dem durch pEX1 codierten ß-Gal als Kontrollprotein. Das Fusionsprotein wurde aus Zellextrakten angereichert und zur Immunisierung von Kaninchen verwendet. Es zeigte sich, daß das Serum, welches nach dreimaliger Immunisierung von jeweils ca. 100 µg mit Komplettem / inkomplettem Freundschem Adjuvans erhalten worden war, in Western blot-Analysen mit einem Protein von ca. 58 000 Dal reagierte, wenn Proteinextrakt aus HCMV-infizierten Zellen oder wenn gereinigte HCMV-Partikel aufgetrennt worden waren. Das Antiserum reagierte hingegen nicht bei aufgetrennten Proteinextrakten aus nicht-infizierten Zellen (Kontrolle). Somit konnte gezeigt werden, daß der in E.coli exprimierte gp 58-Anteil antigene Determinanten trägt, die in der Lage sind, Antikörper zu induzieren, die authentisches gp 58 erkennen. Diese Antikörper sind neutralisiert.

Patentansprüche:

1. Immunogene Proteine des Glykoprotein A-Komplexes des humanen Cytomegalovirus (HCMV), erhalten durch Expression des Gens, das in dem offenen Leserahmen im HindIII-F-Fragment des HCMV-Genoms, Stamm Ad 169, liegt.

2. Hauptglykoprotein und immunogene Bestandteile des HCMV, erhalten durch Expression des Gens, das im HindIII-F-Fragment des HCMV-Genoms, Stamm Ad 169, liegt, in Bakterien, Hefen oder animalen Zellen.

3. Hauptglykoprotein vom Molgewicht von etwa 58 kd aus dem Glykoprotein A-Komplex von HCMV, Stamm Ad 169, erhalten durch Expression des Gens, das auf dem 1,3 kb PstI-EcoRV-Fragment aus dem HindIIIF-Fragment liegt, sowie immunogene Teile dieses Proteins.

4. Verfahren zur Herstellung immunogener Proteine des Glykoprotein A-Komplexes des HCMV, dadurch gekennzeichnet, daß man das Gen ganz oder teilweise zur Expression bringt, das in dem offenen Leserahmen des HindIII-F-Fragments aus dem Genom des Stammes Ad 169 liegt.

5. Verfahren nach Anspruch 4, dadurch gekennzeichnet, daß man aus dem HindIII-F-Fragment ein 3,1 kb PstI-EcoRV-Fragment ganz oder teilweise einsetzt.

6. Verfahren nach Anspruch 4, dadurch gekennzeichnet, daß man aus dem HindIII-F-Fragment ein 1,3 kb PstI-EcoRV-Fragment ganz oder teilweise einsetzt.

7. Verfahren nach einem oder mehreren der vorhergehenden Ansprüche, dadurch gekennzeichnet, daß die Expression in Bakterien, Hefen oder animalen Zellen erfolgt.

8. Verfahren nach Anspruch 7, dadurch gekennzeichnet, daß die Expression in Bakterien erfolgt.

9. Verfahren nach Anspruch 7, dadurch gekennzeichnet, daß die Expression in Hefezellen, tierischen oder menschlichen Zellen erfolgt.

10. Verwendung der Proteine nach den Ansprüchen 1 bis 3 bzw. der nach den Ansprüchen 4 bis 9 erhaltenen Proteine als Diagnostika.

11. Verwendung der Proteine nach den Ansprüchen 1 bis 3 bzw. der nach den Ansprüchen 4 bis 9 erhaltenen Proteine zur Herstellung von Antikörpern.

12. Verwendung der Proteine nach den Ansprüchen 1 bis 3 bzw. der nach den Ansprüchen 4 bis 9 erhaltenen Proteine zur Herstellung von Impfstoffen.

Patentansprüche Griechenland, Österreich und Spanien:

1. Verfahren zur Herstellung immunogener Proteine des Glykoprotein A-Komplexes des humanen Cytomegalovirus (HCMV), dadurch gekennzeichnet, daß man das Gen ganz oder teilweise zur Expression bringt, das in dem offenen Leserahmen des HindIII-F5 Fragments aus dem Genom des Stammes Ad 169 liegt.

2. Verfahren nach Anspruch 1, dadurch gekennzeichnet, daß man aus dem HindIII-F-Fragment ein 3,1 kb PstI-EcoRV-Fragment ganz oder teilweise einsetzt.

3. Verfahren nach Anspruch 1, dadurch gekennzeichnet, daß man aus dem HindIII-F-Fragment ein 1,3 kb PstI-EcoRV-Fragment ganz oder teilweise einsetzt.

4. Verfahren nach einem oder mehreren der vorhergehenden Ansprüche, dadurch gekennzeichnet, daß die Expression in Bakterien, Hefen oder animalen Zellen erfolgt.

5. Verfahren nach Anspruch 4, dadurch gekennzeichnet, daß die Expression in Bakterien erfolgt.

6. Verfahren nach Anspruch 4, dadurch gekennzeichnet, daß die Expression in Hefezellen, tierischen oder menschlichen Zellen erfolgt.

0252302

Nummer der Anmeldung

**Europäisches Patentamt**

**EUROPÄISCHER RECHERCHENBERICHT**

EP  87 10 8169

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl 4) |
|---|---|---|---|
| X | CHEMICAL ABSTRACTS, Band 103, 1985, Seite 379, Zusammenfassung Nr. 119448n, Columbus, Ohio, US; L. PEREIRA: "Glycoproteins specified by human cytomegalovirus", & HERPESVIRUSES 1985, 3, 383-404 * Zusammenfassung * | 1-11 | C 12 N   15/00 // A 61 K   39/245 G 01 N   33/569 |
| | --- | | |
| Y | EP-A-0 180 288   (INSTITUT MERIEUX) * Insgesamt * | 1-11 | |
| | --- | | |
| Y | PROC. NATL. ACAD. SCI. USA, Band 82, Februar 1985, Seiten 1266-1270; E.S. MOCARSKI et al.: "Precise localization of genes on large animal virus genomes: use of lambdagt11 and monoclonal antibodies to map the gene for a cytomegalovirus protein family" * Insgesamt * | 1-11 | RECHERCHIERTE SACHGEBIETE (Int Cl.4) C 12 N G 01 N A 61 K C 12 P |
| | --- | | |
| P,X | CHEMICAL ABSTRACTS, Band 105, 1986, Seite 162, Zusammenfassung Nr. 73525w, Columbus, Ohio, US; M. MACH et al.: "Mapping of the major glycoprotein gene of human cytomegalovirus", & J. GEN. VIROL. 1986, 67(7), 1461-7 * Zusammenfassung * | 1-11 | |
| | ---                     -/- | | |

Der vorliegende Recherchenbericht wurde für alle Patentansprüche erstellt.

| Recherchenort DEN HAAG | Abschlußdatum der Recherche 29-07-1987 | Prüfer SKELLY J.M. |
|---|---|---|

**Europäisches Patentamt**

## EUROPÄISCHER RECHERCHENBERICHT

### EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl 4) |
|---|---|---|---|
| P,X | THE EMBO JOURNAL, Band 5, Nr. 11, 1986, Seiten 3057-3063, IRL Press LTD, Oxford, GB; M.P. CRANAGE et al.: "Identification of the human cytomegalovirus glycoprotein B gene and induction of neutralizing antibodies via its expression in recombinant vaccinia virus" * Insgesamt * | 1-11 | |
| D,A | VIROLOGY, Band 139, 1984, Seiten 73-86, Academic Press Inc.; L. PEREIRA et al.: "Polymorphism of human cytomegalovirus glycoproteins characterized by monoclonal antibodies" * Seite 76, Spalte 2, Zeilen 1-26; Seite 79, Spalte 1, Zeilen 16-28; Spalte 2, Zeilen 1-27; Seite 80, Tabelle I; Seite 84, Spalte 1; Seite 84, Spalte 2, Zeilen 1-46 * | 1-11 | |
| A | PROC. NATL. ACAD. SCI. USA, Band 81, August 1984, Seiten 4965-4969; H. PANDE et al.: "Cloning and physical mapping of a gene fragment coding for a 64-kilodalton major late antigen of human cytomegalovirus" | | RECHERCHIERTE SACHGEBIETE (Int. Cl 4) |

--- -/-

Der vorliegende Recherchenbericht wurde für alle Patentansprüche erstellt.

| Recherchenort DEN HAAG | Abschlußdatum der Recherche 29-07-1987 | Prüfer SKELLY J.M. |
|---|---|---|

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82

**Europäisches Patentamt**

# EUROPÄISCHER RECHERCHENBERICHT

EP 87 10 8169

| | EINSCHLÄGIGE DOKUMENTE | | Seite 3 |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int Cl 4) |
| A | VIROLOGY, Band 132, 1984, Seiten 325-338, Academic Press Inc.; B. NOWAK et al.: "Characterization of monoclonal antibodies and polyclonal immune sera directed against human cytomegalovirus virion proteins"<br><br>--- | | |
| A | VIROLOGY, Band 128, 1983, Seiten 391-406, Academic Press Inc.; W. GIBSON: "Protein counterparts of human and simian cytomegaloviruses"<br><br>----- | | |
| | | | RECHERCHIERTE SACHGEBIETE (Int Cl 4) |

Der vorliegende Recherchenbericht wurde für alle Patentansprüche erstellt.

| Recherchenort | Abschlußdatum der Recherche | Prüfer |
|---|---|---|
| DEN HAAG | 29-07-1987 | SKELLY J.M. |